(19) Europäisches Patentamt
European Patent Office
Office européen des brevets

(11) Veröffentlichungsnummer: **0 219 452 A1**

(12) # EUROPÄISCHE PATENTANMELDUNG

(21) Anmeldenummer: 86810398.7

(22) Anmeldetag: 03.09.86

(51) Int. Cl.[4]: C 07 D 331/02
C 08 K 5/45, C 10 M 135/34

(30) Priorität: 09.09.85 US 773723

(43) Veröffentlichungstag der Anmeldung: 22.04.87 Patentblatt 87/17

(84) Benannte Vertragsstaaten: BE CH DE FR GB IT LI

(71) Anmelder: CIBA-GEIGY AG
Klybeckstrasse 141
CH-4002 Basel(CH)

(72) Erfinder: Steinberg, David H.
2216 Wilson Avenue
New York New York 10469(US)

(72) Erfinder: Cortolano, Frank
3 East Maple Street
Valhalla New York 10595(US)

(54) **Verfahren zur Herstellung von schwefelhaltigen Stabilisatoren mit einer sterischgehinderten Phenolgruppe.**

(57) Verbindungen der Formel I

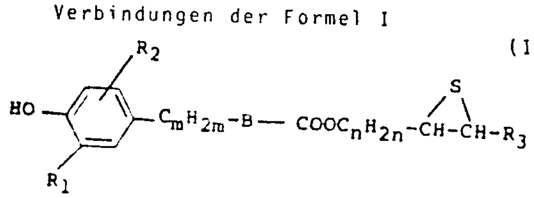

(I)

worin $R_1$ und $R_2$ unabhängig voneinander Alkyl mit 1 bis 6 Kohlenstoffatomen, Cycloalkyl mit 5 bis 8 Kohlenstoffatomen, Allyl, Methallyl, Aralkyl mit 7 bis 9 Kohlenstoffatomen, Phenyl, 1- oder 2-Naphthyl oder Alkaryl mit 7 bis 9 Kohlenstoffatomen sowie $R_2$ ferner Wasserstoff, $R_3$ Wasserstoff, Alkyl mit 1 bis 6 Kohlenstoffatomen oder gegebenenfalls durch ein oder zwei Chloratome und/oder eine oder zwei Methylgruppen substituiertes Phenyl, B ein Heteroatom, die Gruppe $-OC(O)-$ oder $-OC(O)-CH_2CH_2-$ oder eine direkte Bindung, m eine ganze Zahl von 0 bis 6 und n eine ganze Zahl von 1 bis 6 bedeuten, werden durch Reaktion eines Thioglycerinderivats mit einer eine sterisch gehinderte Phenolfuncktion enthaltenden Carbonsäure in Gegenwart einer Säure hergestellt und als Antioxidantien und Hitzestabilisatoren für Schmieröle, Kunststoffe, Harze und sonstige organische Substrate verwendet.

EP 0 219 452 A1

# Verfahren zur Herstellung von schwefelhaltigen Stabilisatoren mit einer sterischgehinderten Phenolgruppe

Vorliegende Erfindung betrifft ein Verfahren zur Herstellung von Thiiranverbindungen, die Verwendung solcher Verbindungen als Stabilisatoren für organische Polymere und Schmiermittel sowie die neuen Thiirane.

Eine Thiiranfunktion enthaltende, sterisch gehinderte Phenole sind bekannte Verbindungen und beispielsweise in U.S. Patent 3 992 420 beschrieben. Diese Verbindungen werden durch Reaktion der entsprechenden Oxiranderivate mit Thioharnstoff oder einem Alkalithiocyanat in einem Lösungsmittel hergestellt, wobei das Oxiran aus den entsprechenden Olefinen durch Umsetzung mit Percarbonsäuren oder aus Alkaliphenoxiden und Epichlorhydrinen hergestellt wird. Bei diesen Methoden handelt es sich um zweistufige Verfahren und die Verwendung mehrerer Zwischenprodukte und Reagenzien. Zudem lassen die nach diesen Verfahren erzielten Gesamtausbeuten und die Reinheit der entstandenen Endprodukte in vielen Fällen etwas zu wünschen übrig.

Weitere Thiiranverbindungen ohne die sterisch gehinderte Phenolgruppe sowie Verfahren zu deren Herstellung sind aus U.S. Patenten 3 359 298 und 3 404 158, Lautenschlaeger u.a., J. Org. Chem. 34 (12), 3991-8 (1969) und Zh. Org. Khim. 12 (3), 562-565 (1976), bekannt.

Gegenstand vorliegender Erfindung ist ein Verfahren zur Herstellung von Thiiranverbindungen der Formel I

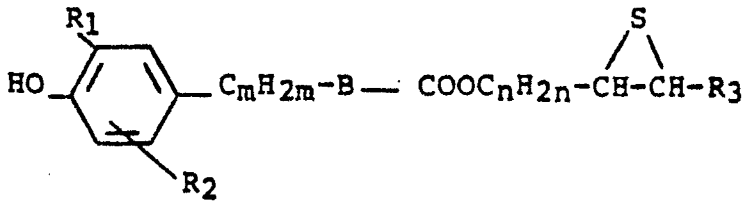

(I),

worin $R_1$ und $R_2$ unabhängig voneinander Alkyl mit 1 bis 6 Kohlenstoffatomen, Cycloalkyl mit 5 bis 8 Kohlenstoffatomen, Allyl, Methallyl, Aralkyl mit 7 bis 9 Kohlenstoffatomen, Phenyl, 1- oder 2-Naphthyl oder Alkaryl mit 7 bis 9 Kohlenstoffatomen sowie $R_2$ ferner Wasserstoff, $R_3$ Wasserstoff, Alkyl mit 1 bis 6 Kohlenstoffatomen oder gegebenenfalls durch ein oder zwei Chloratome und/oder eine oder zwei Methylgruppen substituiertes Phenyl, B ein Heteroatom, die Gruppe $-OC(O)-$ oder $-OC(O)-CH_2CH_2-$ oder eine direkte Bindung, m eine ganze Zahl von 0 bis 6 und n eine ganze Zahl von 1 bis 6 bedeuten, welches dadurch gekennzeichnet ist, dass man eine Verbindung der Formel II

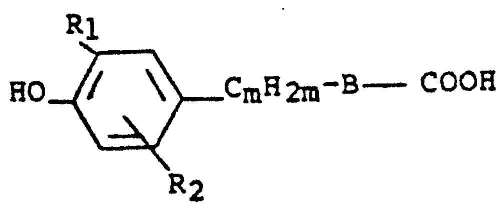

(II)

in Gegenwart einer katalytischen Menge Säure in Gegenwart oder Abwesenheit eines inerten organischen Lösungsmittels mit einem Thioglycerin der Formel III

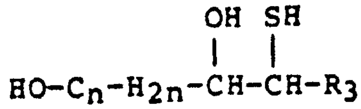

(III),

worin $R_1$, $R_2$, $R_3$, B, m und n die oben angegebenen Bedeutungen haben, zur Reaktion bringt.

In der Definition der Verbindungen der Formel I können $R_1$, $R_2$ und $R_3$ Alkyl mit 1 bis 6 Kohlenstoffatomen wie beispielsweise Methyl, Ethyl, Propyl, Isopropyl, n-Butyl, sek-Butyl, tert-Butyl, Amyl, sek-Amyl, tert-Amyl, Hexyl oder 1,1-Dimethylbutyl sein. Als $R_1$ steht Alkyl vorzugsweise

für Isopropyl, sek-Butyl, tert-Butyl, Isoamyl und 1,1-Dimethylbutyl sowie besonders bevorzugt für tert-Butyl. Als $R_2$ bedeutet Alkyl vorzugsweise Methyl und tert-Butyl.

Als $R_1$ und $R_2$ kann auch eine Cycloalkylgruppe mit 5 bis 8 Kohlenstoffatomen vorliegen, wie beispielsweise Cyclopentyl, Cyclohexyl, α-Methylcyclohexyl oder Cycloheptyl, oder eine Aralkylgruppe mit 7 bis 9 Kohlenstoffatomen wie beispielsweise Benzyl, α-Phenylethyl oder α,α-Dimethylbenzyl. Cyclohexyl und Benzyl sind bevorzugt.

$R_1$ und $R_2$ können auch Alkaryl mit 7 bis 9 Kohlenstoffatomen darstellen, wie beispielsweise o-, m- oder p-Tolyl, 2,3-, 2,4-, 2,5-, 2,6-, 3,4- oder 3,5-Dimethylphenyl oder 2,3,4-, 2,3,5-, 2,3,6-, 2,4,5-, 2,4,6-, 2,5,6- oder 3,4,5-Trimethylphenyl. Für $R_1$ und $R_2$ als Alkaryl werden o-, m- und p-Tolyl bevorzugt.

B kann ein Heteroatom wie beispielsweise -O-, -S- und -NH- sein.

Vorzugsweise ist m 2 und n 1.

Bedeutet $R_3$ durch ein oder zwei Chloratome und/oder Methylgruppen substituiertes Phenyl, so kann dies beispielsweise p-Chlor- oder p-Methylphenyl sowie 2-Methyl-5-chlorphenyl sein.

Solche Verbindungen der Formel I sind bevorzugt, in denen $R_1$ und $R_2$ unabhängig voneinander Alkyl mit 1 bis 4 Kohlenstoffatomen, Cycloalkyl oder α-Methylbenzyl sowie $R_2$ ferner Waserstoff, $R_3$ Wasserstoff oder Methyl, B eine direkte Bindung, m die Zahl 2 und n 1 bedeuten.

Besonders bevorzugte Verbindungen der Formel I sind solche, in denen $R_1$ tert-Butyl, $R_2$ in ortho-Stellung zur Hydroxylgruppe Wasserstoff, Methyl oder tert-Butyl, $R_3$ Wasserstoff, B eine direkte Bindung, m 2 und n 1 bedeuten.

Zur Erläuterung sind einige Beispiele für Verbindungen der Formeln II und III angeführt. Diese Verbindungen sind allgemein handelsüblich oder können nach bekannten präparativen Methoden hergestellt werden.

Verbindungen der Formel II:

3,5-Dimethyl-4-hydroxyphenylpropionsäure, 3-Methyl-5-tert -

butyl-4-hydroxyphenylpropionsäure, 3,5-Diisopropyl-4-hydroxyphenylpropionsäure, 3,5-Di-(tert-butyl)-4-hydroxyphenylpropionsäure, 3,5-Di-tert-pentyl-4-hydroxyphenylpropionsäure, 3,5-Di-(tert-butyl)-phenylessigsäure, 3,5-Di-tert-butylbenzoesäure, 3,5-Di-(tert-butyl)benzylthioessigsäure, β-[3,5-Di-(tert-butyl)benzylthio]-propionsäure und 3,5-Di-(tert-butyl)benzyloxyessigsäure.

Verbindungen der Formel III:
1-Thioglycerin und 3-Mercapto-3-methyl-1,2-propandiol.

Das vorliegende Verfahren wird so durchgeführt, dass man eine Verbindung der Formel II und eine Verbindung der Formel III in Gegenwart einer Säure sowie in Abwesenheit oder Gegenwart eines organischen Lösungsmittels vermischt.

Die Reaktion kann bei verschiedenen Temperaturen erfolgen, zweckmässig im Temperaturbereich von 20°C bis 200°C, vorzugsweise von 50° bis 150°C und besonders bevorzugt von 80° is 110°C. Sie lässt sich bei Atmosphärendruck oder erhöhtem Druck durchführen.

Als inerte organische Lösungsmittel kommen beispielsweise aliphatische oder aromatische Kohlenwasserstoffe wie Hexan, Heptan, Cyclohexan, Benzol oder Alkyl-, Alkoxy- oder Halogen-substituiertes Benzol, z.B. Toluol, Xylole, Anisole, Chlorbenzol oder Dichlorbenzol, Nitrobenzol, Ketone wie Cyclohexanon, Ether wie Tetrahydrofuran oder Dioxan, Glykolether wie Ethylenglykoldimethyl- oder -diethylether, Amide wie Dimethylformamid, Dimethylacetamid oder N-Methylpyrrolidon, Nitrile wie Acetonitril sowie Dimethylsulfoxid oder Sulfolan in Betracht. Gemische der oben erwähnten Lösungsmittel sind ebenfalls verwendbar. Toluol, Xylol oder halogen-substituiertes Benzol werden als Lösungsmittel bevorzugt.

Das erfindungsgemässe Verfahren wird in Gegenwart einer Säure durchgeführt. Beispiele für geeignete Säuren sind aliphatische oder aromatische Carbonsäuren oder Sulfonsäuren, z.B. Ameisensäure, Essigsäure, Propionsäure, Oxalsäure, Benzoesäure, Methansulfonsäure, Benzolsulfonsäure oder p-Toluolsulfonsäure. Zu weiteren geeigneten Säuren zählen Mineralsäuren wie Salzsäure, Schwefelsäure oder Phosphorsäure.

Im erfindungsgemässen Verfahren kann man die starke Säure in einer katalytischen Menge von vorzugsweise 0,001 bis 0,20 Mol und besonders bevorzugt 0,02 bis 0,10 Mol, bezogen auf ein Mol der Verbindung der Formel II, einsetzen. Die Verbindungen der Formel II und III werden in stöchiometrischen Mengen eingesetzt, doch wirkt sich ein Überschuss der Verbindung der Formel III häufig vorteilhaft auf die Ausbeute aus.

Das im Verlauf der Reaktion gebildete Wasser kann durch azeotrope Destillation kontinuierlich entfernt werden. Die nach dem erfindungsgemässen Verfahren erhaltenen Produkte der Formel I lassen sich nach üblichen Methoden wie Dekantieren, Extraktion oder Destillation, vorzugsweise bei vermindertem Druck, gewinnen. Je nach der bestimmungsgemässen Verwendung kann es vorteilhaft sein, das erhaltene Rohprodukt einzusetzen oder es durch Destillation oder Kristallisation aus einem organischen Lösungsmittel zu reinigen.

Zur Umsetzung einer Verbindung der Formel II mit einer Verbindung der Formel III kann man alle Komponenten bei niedrigen Temperaturen im Reaktionsgefäss vorlegen und dann auf die gewünschte Reaktionstemperatur erhitzen oder die Komponenten jeweils einzeln innerhalb des angegebenen Reaktionstemperaturbereichs zuzusetzen. Eine bevorzugte Ausführungsform der Reaktion besteht darin, dass man die Verbindung der Formel II und den sauren Katalysator im Reaktionsgefäss vorlegt und dann die Verbindung der Formel III bei der gewünschten Reaktionstemperatur zusetzt. Eine weitere Möglichkeit besteht darin, dass man die Verbindungen der Formeln II und III gleichzeitig mit der Säure dazugibt. Ferner ist es möglich, das erfindungsgemässe Verfahren chargenweise oder kontinuierlich durchzuführen.

Gegenstand vorliegender Erfindung sind ferner neue Verbindungen der Formel IV

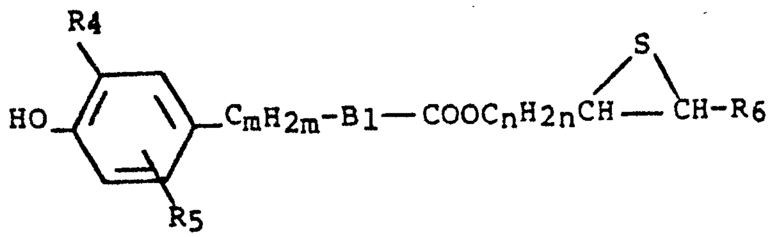

(IV),

worin $R_4$ und $R_5$ unabhängig voneinander Alkyl mit 1 bis 6 Kohlenstoffatomen, Cycloalkyl mit 5 bis 8 Kohlenstoffatomen, Allyl, Methallyl, Aralkyl mit 7 bis 9 Kohlenstoffatomen sowie $R_5$ ferner Wasserstoff, m eine ganze Zahl von 0 bis 6, n eine ganze Zahl von 1 bis 6, $B_1$ ein Heteroatom, die Gruppe -OC(O)- oder $-OC(O)-CH_2-CH_2-$ oder eine direkte Bindung bedeutet und $R_6$, falls $B_1$ eine direkte Bindung ist, gegebenenfalls durch ein oder zwei Chloratome und/oder durch eine oder zwei Methylgruppen substituiertes Phenyl oder, falls $B_1$ ein Heteroatom, die Gruppe OC(O)- oder die Gruppe $-OC(O)-CH_2-CH_2-$ ist, $R_6$ Wasserstoff, Alkyl mit 1 bis 6 Kohlenstoffatomen oder gegebenenfalls durch ein oder zwei Chloratome und/oder eine oder zwei Methylgruppen substituiertes Phenyl bedeutet.

Die erfindungsgemässen Verbindungen der Formeln I und IV sind bei der Stabilisierung organischer Materialien wie Kunststoffen, Polymeren und Harzen, mineralischen und synthetischen Flüssigkeiten wie Schmierölen, Umlaufölen usw. gegen oxidativen, thermischen und aktinischen Abbau besonders wirksam.

Als Substrate, bei denen die erfindungsgemässem Verbindungen der Formeln I und IV besonders nützlich sind, kommen Polyolefine wie Polyethylen und Polypropylen, Polystyrol einschliesslich schlagzähem Polystyrol, ABS-Harz, SBR, Isopren sowie Naturkautschuk, Polyester einschliesslich Polyethylenterephthalat und Polybutylenterephthalat einschliesslich Copolymeren, sowie Schmieröle wie solche, die sich von Mineralöl ableiten, in Betracht.

Zu stabilisierbaren Polymeren gehören allgemein:

1. Polymere von Mono- und Diolefinen, beispielsweise Polyethylen (das gegebenenfalls vernetzt sein kann), Polypropylen, Polyisobutylen, Polybuten-1, Polymethylpenten-1,

Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivten, wie z.B. Styrol-Butadien, Styrol-Acrylnitril, Styrol-Ethylmethacrylat, Styrol-Butadien-Ethylacrylat, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Propfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen,

Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamid und Polyacrylnitril.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten.

13. Polyphenylenoxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte (Polyisocyanate, Polyole oder Präpolymere).

15. Polyamide und Copolyamide, die sich von Diaminen und Dicarbonsäuren und/oder Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalat, Poly-m-phenylenisophthalamid, sowie deren Copolymere mit Polyethern, wie z.B. mit Polyethylenglykol, Polypropylenglykol oder Polytetramethylenglykol.

16. Polyharnstoffe, Polyimide und Polyamid-imide.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylcyclohexanterephthalat, Poly-[2,2-(4-hydroxyphenyl)-propan]-terephthalat, Polyhydroxybenzoate sowie Block-Polyether-ester, die sich von Polyethern mit Hydroxylendgruppen ableiten.

18. Polycarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidhrze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate bzw. die Celluloseether, wie Methylcellulose.

27. Mischungen (Polyblends) der vorgenannten Polymeren, wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische und pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Weichmacher für Polymere oder als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die erfindungsgemässen Verbindungen der Formeln I und IV werden im allgemeinen zu etwa 0,01 bis etwa 5 Gew.-% der zu stabilisierenden Zusammensetzung eingesetzt, doch kann dies je nach dem jeweiligen Substrat und der Anwendung schwanken. Ein bevorzugter Bereich liegt zwischen etwa 0,5 und etwa 2%, und insbesondere 0,1 bis etwa 1%.

Die erfindungsgemässen Stabilisatoren lassen sich leicht nach üblichen Methoden in irgendeiner zweckmässigen Stufe vor der Herstellung von Formkörpern daraus in die organischen Polymeren einarbeiten. Beispielsweise kann man den Stabilisator in trockener Pulverform oder eine Suspension oder Emulsion des Stabilisators mit einer Lösung, Suspension oder Emulsion des Polymeren vermischen. Die dabei erhaltenen, erfindungsgemäss stabilisierten Polymerzusammensetzungen können gegebenenfalls noch verschiedene herkömmliche Zusatzstoffe wie die folgenden enthalten.

1. Antioxidantien

1.1. Alkylierte Monophenole, z.B. 2,6-Di-tert-butyl-4-methylphenol, 2-tert-butyl-4,6-dimethylphenol, 2,6-Di-(tert-butyl)-4-ethylphenol, 2,6-Di-(tert-butyl)-4-n-butylphenol, 2,6-Di-(tert-butyl)-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-(tert-butyl)-4-methoxymethylphenol.

1.2. Alkylierte Hydrochinone, z.B. 2,6-Di-(tert-butyl)-4-methoxyphenol, 2,5-Di-(tert-butyl)hydrochinon, 2,5-Di-(tert-amyl)-hydrochinon, 2,6-Diphenyl-4-octadecyloxyphenol.

1.3. Hydroxylierte Thiodiphenylether, z.B. 2,2'-Thio-bis-(6-tert.butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol).

1.4. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-[6-(α-methylbenzyl)-4-nonylphenol], 2,2'-Methylen-bis-[6-(α,α-dimethylbenzyl)-4-nonylphenol], 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 4,4'-Methylen-bis-(2,6-di-tert-butylphenol), 4,4'-Methylen-bis-(6-tert-butyl-2-methylphenol), 1,1-bis-(5-tert-butyl-4-hydroxy-2-methyl-phenyl)-butan, 2,6-Di-(3-tert-butyl-5-methyl-2-hydroxyben-zyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglykol-bis-[3,3-bis-(3'-tert-butyl-4'-hydroxyphenyl)-butyrat], Di-(3-tert-butyl-4-hydroxy-5-methylphenyl)-dicyclopentadien, Di-[2-(3'-tert-butyl-2'-hydroxy-5'-methyl-benzyl)-6-tert-butyl-4-methyl-phenyl]-terephthalat.

1.5. Benzylverbindungen, z.B. 1,3,5-Tri-(3,5-di-tert-butyl-4-hydroxybenzyl)-2,4,6-trimethylbenzol, Di-(3,5-di-tert-butyl-4-hydroxybenzyl)-sulfid, 3,5-Di-tert-butyl-4-hydroxybenzyl-mercaptoessigsäure-isooctylester, Bis-(4-tert-butyl-3-hydroxy-2,6-dimethylbenzyl)-dithiolterephthalat, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 3,5-Di-(tert-butyl)-4-hydroxybenzyl-phosphonsäure-dioctadecylester, 3,5-Di-(tert-butyl)-4-hydroxybenzyl-phosphonsäure-monoethylester, Calcium-salz.

1.6. Acylaminophenole, z.B. 4-Hydroxy-laurinsäureanilid, 4-Hydroxystearinsäureanilid, 2,4-Bis-octylmercapto-6-(3,5-di-tert-butyl-4-hydroxyanilino)-s-triazin, N-(3,5-Di-tert -butyl-4-hydroxyphenyl)-carbaminsäure-octylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglykol, Thiodiethylenglykol, Diethylenglykol, Triethylenglykol, Pentaerythrit, Tris-hydroxyethyl-isocyanurat, Di-hydroxyethyl-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglykol, Thiodiethylenglycol, Diethylenglykol, Triethylenglykol, Pentaerythrit, Tris-hydroxyethyl-isocyanurat, Di-hydroxyethyl-oxalsäurediamid.

1.9. Amide der β-(3,5-Di-tert.butyl-4-hydroxyphenyl)-propionsäure, wie z.B. N,N'-Bis-(3,5-di-tert-butyl-4-hydroxy phenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert-butyl-, 5'-tert-Butyl-, 5'-(1,1,3,3-Tetramethylbutyl)-, 5-Chlor-3',5'-di-tert-butyl-, 5-Chlor-3'-tert-butyl-5'-methyl-, 3'-sek-Butyl-5'-tert-butyl, 4'-Octoxy-, 3',5'-Di-tert-amyl-, 3',5'-Bis-(α,α-dimethylbenzyl)-Derivat.

2.2. 2-Hydroxybenzophenone, wie z.B., das 4-Hydroxy, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. Phenylsalicylat, 4-tert-Butyl-phenylsalicylat, Octylphenyl-salicylat, Dibenzoylresorcin, Bis-(4-tert-butyl-

benzoyl)-resorcin, Benzoylresorcin, 3,5-Di-(tert-butyl)-4-hydroxybenzoesäure-2,4-di-(tert-butyl)phenylester, 3,5-Di-(tert-butyl)-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyan-β-methyl-p-methoxy-zimtsäuremethylester bzw. -butylester, α-Carbomethoxy-p-methoxy-zimtsäure-methyl-ester, N-(β-Carbomethoxy-β-cyanvinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1 oder der 1:2 Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-di-(tert-butyl)benzylphosphonsäure monoalkylestern, wie vom Methyl-, Ethyl- oder Butylester, Nickelkomplexe von Ketoximen, wie von 2-Hydroxy-4-methylphenylundecylketonoxim, Nickelkomplexe des 1-Phenyl-4-lauroyl-5-hydroxypyrazoles, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert-butyl-4-hydroxybenzylmalonsäure-bis-(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2 2,6,6-Tetramethylpiperidyl)-hexamethylendiamin und tert.Octylamino-2,6-dichlor-1,3,5-s-triazin, Tris-(2,2,6,6-tetramethylpiperidyl)-nitrilotriacetat, Tetrakis-(2,2,6,6-tetramethyl-4-piperidyl)-1,2,3,4-butantetracarbonsäure, 1,1'-[1,2-Ethandiyl-bis-(3,3,5,5-tetramethyl)-piperazinon].

2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5'-di-(tert-butyl)-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-(tert-butyl)-oxanilid, Gemische von o- und p-Methoxy- sowie von o- und p-Ethoxy-disubstituierten Oxaniliden.

3. Metalldesaktivatoren, wie z.B. N,N'-Diphenyloxalsäurediamid, N-Salicylal-N'-salicyloylhydrazin, N,N'-Bis-salicyl-oylhydrazin, N,N'-Bis-(3,5-di-tert-butyl-4-hydroxyphenyl-propionyl)-hydrazin, 3-Salicyloylamino-1,2,4-triazol, Bisbenzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythritdiphosphit, Di-(2,4-di-tert-butylphenyl)-pentaerythritdiphosphit, Tristearyl-sorbit-triphosphit, Tetrakis-(2,4-di-tert-butylphenyl)-4,4'-biphenylen-diphosphonit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Festtsäuren, beispielsweise Ca-Stearat, Zn-Stearat, Mg-Stearat, Na-Ricinoleat und K-Palmitat, Antimonbrenzcatechinat oder Zinkbrenncatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, optische Aufheller, Flammschutzmittel, Antistatika, Treibmittel und Thiosynergisten wie Dilaurylthiodipropionat oder Distearylthiodipropionat.

Die nachfolgenden Beispiele dienen der Erläuterung dieser Erfindung. Dabei sind Teile stets Gewichtsteile, falls nicht anders angegeben.

## Beispiel 1

**Synthese von 3-(3',5'-Di-tert-butyl-4'-hydroxyphenyl)-propionsäure-thiaglycidylester**

a) In einem 500 ml-Dreihalsreaktionskolben legt man 41,76 g (0,15 Mol) 3-(3',5'-Di-tert-butyl-4'-hydroxyphenyl )-propionsäure, 1,43 g (0.0075 Mol) p-Toluolsulfonsäure und 270 ml trockenes Toluol vor, erhitzt das so erhaltene Reaktionsgemisch unter Rühren zum Rückfluss und versetzt tropfenweise im Verlauf einer Stunde unter fortgesetztem Rühren mit 17,84 g (0,165 Mol) Thioglycerin. Das sich dabei bildende Wasser wird kontinuierlich durch azeotrope Destillation entfernt und mittels eines Dean-Stark-Aufsatzes abgefangen. Nach ungefähr 3 1/2 Stunden Reaktion unter Rückfluss ist die Ausgangscarbonsäure praktisch vollständig verbraucht (DSC-Kontrolle). Nach Abkühlen auf Raumtemperatur überführt man das so erhaltene Reaktionsgemisch in einen 1-Liter-Scheidetrichter und wäscht mit 100 ml Wasser. Dies wird dreimal mit 100 ml Wasser wiederholt, wobei der pH am Ende 7,0 beträgt. Die Toluollösung des Produkts wird zweimal mit 100 ml gesättigter Kochsalzlösung gewaschen und 18 Stunden lang über Molekularsieben getrocknet. Entfernung der Siebe durch Filtrieren und Abstreifen des Lösungsmittels bei vermindertem Druck liefert 45,8 g gelben Sirup (87 % Rohausbeute).

b) Man löst 45,8 g Rohprodukt heiss in 250 ml Hexan und filtriert die entstandene Lösung zur Entfernung von ungefähr 0,5 g unlöslichem Produkt. Dieses Hexanfiltrat wird dann heiss ungefähr 30 Minuten lang mit 5,0 g Entfärbungsmittel Fulment® 237 behandelt. Dann filtriert man heiss, lässt auf Raumtemperatur abkühlen und streift im Vakuum ab. Der Rückstand (42,25 g) wird in 150 ml Heptan weideraufgelöst und nochmals 30 Minuten lang mit 4,2 g Fulment® 237 unter Rühren und Erhitzen behandelt.

Das entstandene Gemisch wird warm filtriert, auf

Raumtemperatur abkühlen gelassen und angeimpft (mit authentischen A18-106-Impfkristallen). Unter schwachem Rühren kristallisiert das Produkt langsam ohne Ölbildung aus. Nach Filtrieren erhält man 18,9 g Produkt der Formel

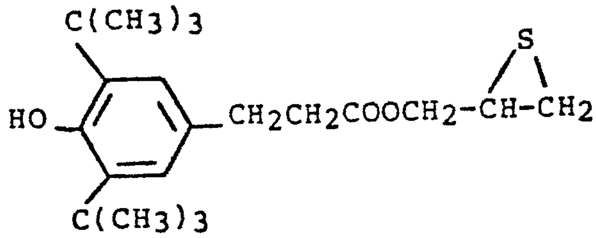

mit Schmelzpunkt 74-79°C und der folgenden Mikroanalyse:
Berechnet für $C_{20}H_{30}O_3S$:

| | | | |
|---|---|---|---|
| | C % 68,53; | H % 8,63; | S % 9,15 |
| Gefunden | C % 68,3; | H % 8,8; | S % 8,9. |

Beispiel 2

Die gemäss Beispiel 1b erhaltene Verbindung wird nach dem folgenden Öloxidationstest als Stabilisator und Antioxdans in einem Schmieröl bewertet. Dieser Test (Normalausführung nach ASTM 2272, Oxidationstest in rotierender Bombe) wird wie folgt durchgeführt. Man oxidiert eine Ölprobe aus 50 g eines paraffinischen Mineralöls 150 N in einem Glasgefäss in einer Sauerstoffatmosphäre zusammen mit 5 ml destilliertem Wasser und einer katalytisch aktiven, mit Petroläther gewaschenen Kupferspirale, unter Zusatz von 0,25 Gew.-% des Stabilisators nach Beispiel 1b.

Das Glasgefäss befindet sich in einer Edelstahlbombe mit Manometer. Die Bombe rotiert um ihre Achse mit 100 Umdrehungen pro Minute unter einem Winkel von 30° zur Horizontalen in einem 150°C heissen Ölbad. Der Sauerstoffdruck beträgt anfänglich 6 bar vor dem Erhitzen, steigt auf genau 14 bar bei 150°C und bleibt konstant bis zum Beginn der Oxidation. Der Test ist beendet, wenn der Druck um 1,7 bar abgefallen ist. Die Zeit in Minuten wird notiert.

Ergebnis

| Stabilisator | Minuten bis zum Druckabfall um 1,7 bar |
|---|---|
| - | 25 |
| nach Beispiel 1b | 250 |

Der Zeitzuwachs bis zum Erscheinen des Druckabfalls ist ein Indiz für eine gute stabilisierende Wirkung.

0219452



Patentansprüche:

1. Verfahren zur Herstellung von Verbindungen der Formel I

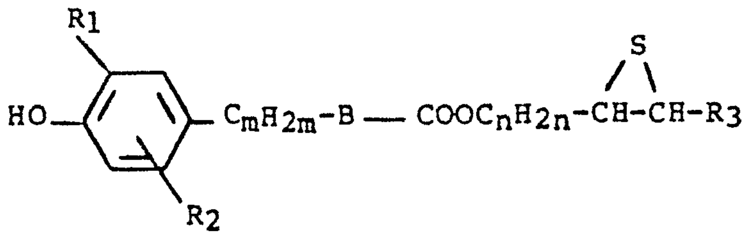

(I),

worin $R_1$ und $R_2$ unabhängig voneinander Alkyl mit 1 bis 6 Kohlenstoffatomen, Cycloalkyl mit 5 bis 8 Kohlenstoffatomen, Allyl, Methallyl, Aralkyl mit 7 bis 9 Kohlenstoffatomen, Phenyl, 1- oder 2-Naphthyl oder Alkaryl mit 7 bis 9 Kohlenstoffatomen sowie $R_2$ ferner Wasserstoff, $R_3$ Wasserstoff, Alkyl mit 1 bis 6 Kohlenstoffatomen oder gegebenenfalls durch Chlor, Methyl oder Chlor und Methyl substituiertes Phenyl, B ein Heteroatom, die Gruppe -OC(O)- oder $-OC(O)-CH_2-CH_2-$ oder eine direkte Bindung, m eine ganze Zahl von 0 bis 6 und n eine ganze Zahl von 1 bis 6 bedeuten, dadurch gekennzeichnet, dass man eine Verbindung der Formel II

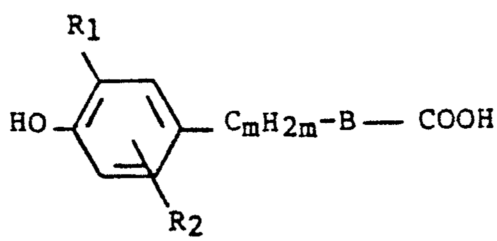

(II)

in Gegenwart einer katalytischen Menge Säure mit einem Thioglycerin der Formel III

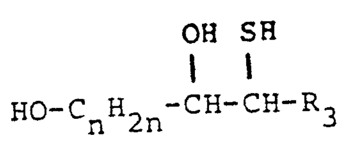

(III),

worin $R_1$, $R_2$, $R_3$, B, m und n die oben angegebenen Bedeutungen haben, zur Reaktion bringt.

2. Verfahren nach Anspruch 1, dadurch gekennzeichnet, dass die Reaktion in einem inerten organischen Lösungsmittel erfolgt.

3. Verfahren nach Anspruch 2, dadurch gekennzeichnet,

dass als organisches Lösungsmittel ein aromatischer Kohlenwasserstoff vorliegt.

4. Verfahren nach Anspruch 3, dadurch gekennzeichnet, dass als Lösungsmittel Toluol vorliegt.

5. Verfahren nach Anspruch 1, dadurch gekennzeichnet, dass die Reaktion im Temperaturbereich von $20^o$ bis $200^oC$ erfolgt.

6. Verfahren nach Anspruch 5, dadurch gekennzeichnet, dass der Temperaturbereich bei $80^o$ bis $110^oC$ liegt.

7. Verfahren nach Anspruch 1, dadurch gekennzeichnet, dass als Säure eine aliphatische oder aromatische Carbonsäure oder Sulfonsäure oder eine Mineralsäure vorliegt.

8. Verfahren nach Anspruch 1 zur Herstellung von Verbindungen der Formel I, worin $R_1$ und $R_2$ unabhängig voneinander Alkyl mit 1 bis 4 Kohlenstoffatomen, Cycloalkyl oder α-Methylbenzyl sowie $R_2$ ferner Wasserstoff, $R_3$ Wasserstoff oder Methyl, B eine direkte Bindung, m die Zahl 2 und n 1 bedeuten.

9. Verfahren nach Anspruch 8, worin $R_1$ tert-Butyl, $R_2$ in ortho-Stellung zur Hydroxylgruppe Wasserstoff, Methyl oder tert-Butyl, $R_3$ Wasserstoff, B eine direkte Bindung, m 2 und n 1 bedeuten.

10. Verbindungen der Formel IV

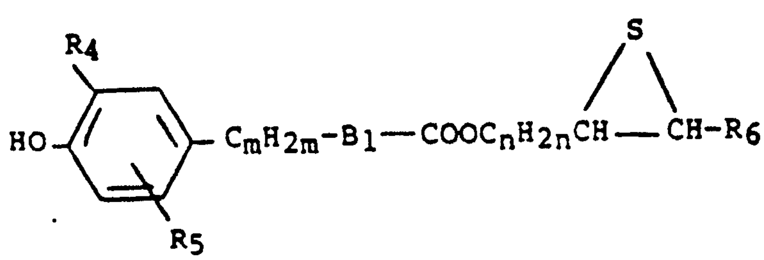

(IV),

worin $R_4$ und $R_5$ unabhängig voneinander Alkyl mit 1 bis 6 Kohlenstoffatomen, Cycloalkyl mit 5 bis 8 Kohlenstoffatomen, Allyl, Methallyl, Aralkyl mit 7 bis 9 Kohlenstoffatomen sowie $R_5$ ferner Wasserstoff, m eine ganze Zahl von 0 bis 6, n eine ganze Zahl von 1 bis 6, $B_1$ ein Heteroatom, die Gruppe -OC(O)- oder $-OC(O)-CH_2-CH_2-$ oder eine direkte Bindung und $R_6$, falls $B_1$ eine direkte Bindung ist, gegebenenfalls durch Chlor, Methyl oder Chlor und Methyl

substituiertes Phenyl bzw., falls $B_1$ ein Heteroatom, die Gruppe -OC(O)- oder die Gruppe $-OC(O)-CH_2-CH_2-$ ist, Wasserstoff, Alkyl mit 1 bis 6 Kohlenstoffatomen oder gegebenenfalls durch Chlor, Methyl oder Chlor und Methyl substituiertes Phenyl bedeuten.

11. Stoffzusammensetzung, dadurch gekennzeichnet, dass sie aus einem oxidativem, thermischem oder aktinischem Abbau unterliegenden organischen Material besteht, das mit einer wirksamen Menge einer Verbindung der Formel IV nach Anspruch 10 stabilisiert ist.

12. Zusammensetzung nach Anspruch 11, dadurch gekennzeichnet, dass als organisches Material ein Schmieröl vorliegt.

13. Verfahren zum Stabilisieren organischen Materials gegen oxidativen, thermischen und aktinischen Abbau, dadurch gekennzeichnet, dass man eine wirksame stabilisierende Menge einer Verbindung der Formel IV nach Anspruch 10 in das besagte organische Material einarbeitet.

0219452

Europäisches Patentamt

# EUROPÄISCHER RECHERCHENBERICHT

Nummer der Anmeldung

EP 86810398.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | US - A - 3 992 420 (LIND et al.)<br>* Patentanspruch 1; Spalten1-4*<br>---- | 1,10-13 | C 07 D 331/02<br>C 08 K 5/45<br>C 10 M 135/34 |
| | | | RECHERCHIERTE SACHGEBIETE (Int. Cl.4) |
| | | | C 07 D 331/00<br>C 08 K 5/00<br>C 10 M 135/00 |

Der vorliegende Recherchenbericht wurde für alle Patentansprüche erstellt.

| Recherchenort | Abschlußdatum der Recherche | Prüfer |
|---|---|---|
| WIEN | 12-12-1986 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82